# EUROPEAN PATENT SPECIFICATION

(11) **EP 2 930 235 B1**
(45) Date of publication and mention of the grant of the patent: **10.03.2021**
(21) Application number: 13860196.8
(22) Date of filing: 22.07.2013
(51) Int. Cl.: C12N 1/04, C12N 1/20, C12N 1/38

(54) **LACTOBACILLUS HAVING ABILITY TO INDUCE IL-12 PRODUCTION, AND METHOD FOR CULTURING SAME**
LACTOBACILLUS MIT FÄHIGKEIT ZUR INDUZIERUNG DER IL-12-PRODUKTION UND VERFAHREN ZUR KULTIVIERUNG DAVON
LACTOBACILLUS APTE À INDUIRE LA PRODUCTION D'IL-12, ET SON PROCÉDÉ DE MISE EN CULTURE

(30) Priority: 07.12.2012 JP 2012267793
(43) Date of publication of application: 14.10.2015
(73) Proprietor: Biogenics Korea Co., Ltd., Seocho-gu Seoul 06628 (KR); Bio-Lab Ltd., Saitama 350-1325 (JP); The Japanese Association Of Clinical Research on Supplements, Saitama 350-1243 (JP)
(72) Inventor: KAN, Tatsuhiko, Sayama-shi Saitama 350-1325 (JP); OHWAKI, Makoto, Hidaka-shi Saitama 350-1243 (JP)
(74) Representative: Staeger & Sperling Partnerschaftsgesellschaft mbB
(86) International application number: PCT/KR2013/006528
(87) International publication number: WO 2014/088183

(56) References cited:
- EP-A1- 2 412 797
- JP-A- 2010 132 579
- KR-A- 20070 078 107
- KR-A- 20070 110 115
- KR-A- 20090 024 175
- KR-A- 20110 009 560
- IGARASHI TOSHINORI: "Study of the relationship between changes in lactic acid bacterial cell components and stimulation of IL-12 production under salt-stressed conditions.", BIOSCIENCE, BIOTECHNOLOGY, AND BIOCHEMISTRY 2010, vol. 74, no. 11, 2010, pages 2171-2175, XP002759610, ISSN: 1347-6947
- TOSHIHIRO SASHIHARA ET AL: "Effect of growth conditions of Lactobacillus gasseri OLL2809 on the immunostimulatory activity for production of interleukin-12 (p70) by murine splenocytes", INTERNATIONAL JOURNAL OF FOOD MICROBIOLOGY, vol. 120, no. 3, 1 December 2007 (2007-12-01), pages 274-281, XP055013338, ISSN: 0168-1605, DOI: 10.1016/j.ijfoodmicro.2007.09.003
- FUJIKI TAKASHI ET AL: "Enhanced immunomodulatory activity and stability in simulated digestive juices of Lactobacillus plantarum L-137 by heat treatment.", BIOSCIENCE, BIOTECHNOLOGY, AND BIOCHEMISTRY 2012, vol. 76, no. 5, 7 May 2012 (2012-05-07), pages 918-922, XP002759611, ISSN: 1347-6947
- MASUDA ET AL: "Immunomodulatory effect of halophilic lactic acid bacterium Tetragenococcus halophilus Th221 from soy sauce moromi grown in high-salt medium", INTERNATIONAL JOURNAL OF FOOD MICROBIOLOGY, ELSEVIER BV, NL, vol. 121, no. 3, 1 November 2007 (2007-11-01), pages 245-252, XP022439569, ISSN: 0168-1605, DOI: 10.1016/J.IJFOODMICRO.2007.10.011

## Description

### Technical Field

This invention relates to *Lactobacillus* having an ability to induce IL-12 production, and a method for preparing the same.

### Background Art

*Lactobacillus* sp. is known to have an effect of relieving allergy symptoms or improving immune activities in addition to improvement of intestinal flora. For example, IL-12 is a cytokine which is secreted from antigen-presenting cells such as dendritic cells or macrophages. In this case, IL-12 is a very potent immunostimulator that activates natural killer cells (NK cells), lymphokine-activated killer cells (LAK cells) or killer T cells (CTL cells) which directly attack cancer cells, or enhances production of interferon-γ (IFN-γ). Also, IL-12 has an immunoregulatory function of shifting the Th1/Th2 balance into Th1. *Lactobacillus* sp. is known to have the ability to induce IL-12 production (Patent Document 1). When the *Lactobacillus* sp. is orally ingested, it can directly act on immunocompetent cells taking part in gut immunity to induce the IL-12 production, thereby promoting immunostimulation or mitigation of allergy symptoms.

Meanwhile, a variety of culture mediums are used to culture *Lactobacillus* sp. In this case, culture mediums having rich nutrients, including a yeast extract, peptone, a meat extract, and the like, have been generally used to facilitate the growth of *Lactobacillus* sp. However, the propagation of the *Lactobacillus* sp. is inhibited since pH of the culture medium is reduced due to its metabolites (for example, lactic acid, etc.). Therefore, a neutralization culture in which the pH of the culture medium is adjusted in the vicinity of neutral pH is performed.

Also, the culture medium conditions are known to have an influence on the activities of the *Lactobacillus* sp. For example, Patent Document 2 discloses that a *Lactobacillus* strain having a high immunostimulatory effect is obtained by culturing *Lactobacillus* sp. using a culture medium containing corn steep liquor and a hydrolysate of casein. Further, Patent Document 3 discloses that a *Lactobacillus* strain having a high immunoregulatory effect is obtained by culturing *Lactobacillus* sp. in a culture medium (pH 3.5 to 5.0).

### [Prior-art Documents]

### [Patent Documents]

Patent Document 1: Japanese Patent No. 4621218
Patent Document 2: Japanese Patent Laid-open Publication No. 2004-41099
Patent Document 3: International Publication No. 2007/138993

Further prior art documents relating to the background of the inventions are the publication EP 2 412 797 A and KR 2009 0024175 A. Reference is also made to the following publications of IGARASHI TOSHINORI: "Study of the relationship between chnges in lactic acid bacterial cell components and stimulation of IL-12 production under salt-stressed conditions", TOSHIHIRO SASHIHARA ET AL: "Effect of growth conditions of Lactobacillus gasseri [...]" and of FUJIKI TAKASHI ET AL: "Enhanced immunomodulatory activity and stability in simulated digestive juices of Lactobacillus plantraum L-137 by heat treatment" and the publication of MASUDA ET AL: "Immunomodulatory effect of halophilic lactic acid bacterium Tetragenococcus halophilus Th221 from soy sauce moromi grown in high-salt medium", INTERNATIONAL JOURNAL OF FOOD MICROBIOLOGY, ELSEVIER BV, NL,vol. 121, no. 3, 1 November 2007 (2007-11-01), pages 245-252, XP022439569, ISSN:0168-1605, DOI:10.1016/J.IJFOODMICRO.2007.10.011.

### Summary

### Technical Problem

However, technology of changing the culture medium conditions to enhance the activities of *Lactobacillus* sp. as disclosed in Patent Documents 2 and 3 is rather unfavorable in an aspect of reducing preparation costs so that *Lactobacillus* sp. is cultured to obtain a large volume of the *Lactobacillus* strain, and thus there is no conventional technology compatible to the technology.

Therefore, it is an aspect of the present invention to provide novel technology capable of preparing a large volume of a *Lactobacillus* strain and also enhancing the activity of the *Lactobacillus* strain.

### Solution to Problem

The present inventors have conducted research to solve the above problems, and found that, when a neutralization culture is performed to realize a large volume of *Lactobacillus* sp. to prepare a *Lactobacillus* strain, the activity of the *Lactobacillus* sp. is reduced compared to when the *Lactobacillus* sp. is cultured without adjusting pH, but the activity of the *Lactobacillus* sp. may be recovered or further enhanced by keeping the *Lactobacillus* sp. in an environment which is undesirable to the growth of the *Lactobacillus* sp. at a later stage of the cultivation after the neutralization culture. Therefore, the present invention has been completed based on these facts.

That is, the present invention provides a *Lactobacillus* strain having an ability to induce IL-12 production, and a method for preparing the same, as configured in the claims.

### Advantageous Effects of Invention

According to one exemplary embodiment of the present invention, the activity of *Lactobacillus* sp. reduced due to a neutralization culture, especially, an ability to induce IL-12 production can be recovered or further enhanced by performing a neutralization culture on the *Lactobacillus* sp. to enhance a growth ability of the *Lactobacillus* sp. so as to obtain a large volume of a *Lactobacillus* strain, and keeping the *Lactobacillus* sp. in an environment which is undesirable to the growth of the *Lactobacillus* sp. at a later stage of the cultivation after the neutralization culture. Therefore, it is possible to reduce the preparation cost and also prepare a *Lactobacillus* strain having high activities.

### Brief Description of Drawings

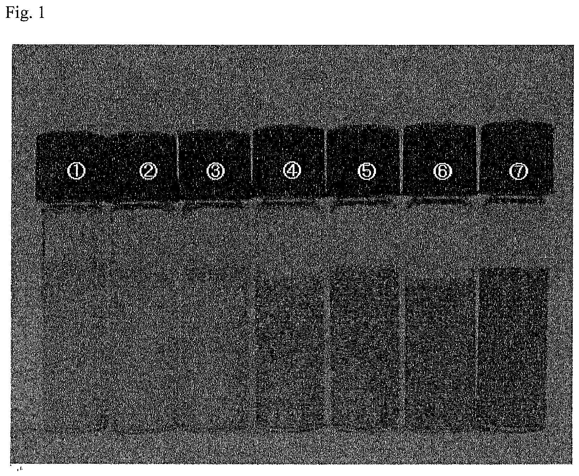

Fig. 1 is an image obtained by comparing staining states of a *Lactobacillus* strain, which is not sterilized after culturing the *Lactobacillus* strain, and staining states of a *Lactobacillus* strain, which is obtained by sterilizing the *Lactobacillus* strain under a predetermined condition and collecting the *Lactobacillus* strain.

### Detailed Description

Among the *Lactobacillus* sp., *L. plantarum* or *L. brevis* isolated from plants is particularly preferably applied since the strain has significant synergic effects as will be described later in Examples.

According to one exemplary embodiment of the present invention, *Lactobacillus* sp. sufficiently grows prior to cultivation to ensure a large volume of a *Lactobacillus* strain. For this purpose, it is necessary to culture the *Lactobacillus* strain in a culture medium suitable for propagation of the *Lactobacillus* strain and simultaneously culture the *Lactobacillus* strain by adding an alkaline chemical to the culture medium to suppress pH reduction caused by metabolites (for example, lactic acid, etc.) of the *Lactobacillus* strain and while adjusting a pH value.

An aqueous solution such as sodium hydroxide, potassium hydroxide, or calcium hydroxide, or ammonia may be used as the alkaline chemical. The pH value is preferably maintained in a range of pH 5.8 to 7.5, more preferably pH 6.0 to 6.8. The pH adjustment is manually performed, but may be easily and accurately performed using a pH stat.

The culture medium is not particularly limited as long as it is suitable for the propagation of the *Lactobacillus* strain. The culture medium suitable for the propagation of the *Lactobacillus* strain may include culture mediums having rich nutrients, including a yeast extract, peptone, a meat extract, and the like. Commercially available culture mediums that may be used herein may also include a "Difco Lactobacilli MRS broth" (trade name, Becton Dickinson & Company, Japan), "MRS Bouillon MERCK" (trade name, Chemicals Co.), etc. Also, when special compositions of the culture medium are required according to the types of *Lactobacillus* sp., desired compositions of the culture medium may be used.

As one typical example of the neutralization culture, when it is assumed that *E. faecalis* is cultured at a starting strain concentration of 1×10⁶ to 1×10⁷ cfu/mL, *E. faecalis* may propagate in a culture medium of "Difco Lactobacilli MRS broth" (trade name, Becton Dickinson & Company, Japan) until the final strain concentration reaches 5×10⁹ to 5×10¹⁰ cfu/mL when the strain is cultured at 35 to 38°C for 16 to 30 hours while maintaining a pH value in a range of pH 6.5 to 6.8.

Also, when it is assumed that *L. plantarum* is cultured at a starting strain concentration of 1×10⁶ to 1×10⁷ cfu/mL, *L. plantarum* may propagate in a culture medium of "Difco Lactobacilli MRS broth" (trade name, Becton Dickinson & Company, Japan) until the final strain concentration reaches 5×10⁹ to 5×10¹⁰ cfu/mL when the strain is cultured at 30 to 35°C for 18 to 40 hours while maintaining a pH value in a range of pH 6.0 to 6.2.

According to one exemplary embodiment of the present invention, it is necessary to apply stress to the strain at a later stage of the cultivation after the neutralization culture. Here, the term "stress" generally refers to all environments which are unfavorable to the growth of *Lactobacillus* sp. Therefore, the activity of the *Lactobacillus* sp. reduced by the above-described neutralization culture, especially an ability to induce IL-12 production may be recovered, or further enhanced.

A method of applying stress may, for example, include the following methods:
(a) cultivating the *Lactobacillus* sp. without adding the alkaline chemical;
(b) cultivating the *Lactobacillus* sp. in a temperature range in which the propagation of the *Lactobacillus* sp. is suppressed;
(c) cultivating the *Lactobacillus* sp. after a salt is added at a concentration of 1% by weight or more in a culture broth and wherein
the stress is selected from the group consisting of [(a) and (c)] or [(a), (b) and (c)]

In operation (a), the *Lactobacillus* sp. is kept under low pH conditions as the pH of the culture medium is reduced by lactic acid produced by the *Lactobacillus* sp., resulting in an environment which is unfavorable for their growth. In this case, it is preferred to culture the *Lactobacillus* sp. until the pH value reaches at least pH 5 or less without adding the alkaline chemical.

In operation (b), an environment unfavorable to the growth of *Lactobacillus* sp. is formed by keeping the *Lactobacillus* sp. at a predetermined temperature. The temperature condition may be properly set to a wide extent according to the *Lactobacillus* sp. used. For example, the temperature condition is set in a temperature range which is preferably shifted from a temperature range suitable for the growth of *Lactobacillus* sp. to a high or low temperature of 3 to 12°C, more preferably 5 to 10°C.

In operation (c), an environment unfavorable to the growth of *Lactobacillus* sp. is formed by keeping the *Lactobacillus* sp. at a high osmotic pressure. A salt such as NaCl is added so that the salt in the culture broth is preferably included at a concentration of 0.5% by weight or more, more preferably 1 to 2% by weight.

According to the present invention, it is also necessary to sterilize the *Lactobacillus* sp. which is completely cultured as described above. Therefore, the self-digestion of the *Lactobacillus* sp. may be prevented to secure the quality stability.

The sterilization method is not particularly limited, and may include a method of culturing *Lactobacillus* sp. in the same manner as practiced in the related art, removing a culture medium by means of filtration, centrifugation or precipitation, collecting the *Lactobacillus* sp., suspending the *Lactobacillus* sp. in water or a buffer to adjust a concentration of the *Lactobacillus* concentrate, and autoclaving the *Lactobacillus* concentrate at 80 to 100°C. Also, even when the *Lactobacillus* sp. is fed into a spray drying apparatus for the purpose of drying and pulverizing the *Lactobacillus* sp., the *Lactobacillus* sp. may be sterilized in this process. Therefore, such a spray drying process may be desirable.

However, as will be described in Examples below, sterilizing the *Lactobacillus* sp. in a culture broth after the cultivation and collecting the *Lactobacillus* sp. result in a higher ability to induce IL-12 production, compared to sterilizing the *Lactobacillus* sp. in a state in which the culture broth is removed after the strain collection. As a result, in one preferred embodiment, the *Lactobacillus* sp. is sterilized in the culture broth after cultivation, and then collected. In this case, for example, the sterilization method may include a method of heat-sterilizing the *Lactobacillus* sp. in the culture broth after the cultivation, removing the culture medium by means of filtration, centrifugation or precipitation, and collecting the *Lactobacillus* sp. Also, a method of removing some of the culture medium by means of filtration, centrifugation or precipitation after the cultivation, preparing a concentrate concentrated 2 to 10 times, subjecting the concentrate to heat sterilization, removing the other culture medium by means of filtration, centrifugation or precipitation, and collecting the *Lactobacillus* sp. is possible. The heat sterilization is preferably performed at 80°C or more, more preferably at 80 to 100°C for 5 to 20 minutes.

However, when the *Lactobacillus* sp. is sterilized in the culture broth after the cultivation as will be described in Examples below, the resulting *Lactobacillus* strain is stained with yellowish brown, and an ability to induce IL-12 production is enhanced when the yellowish brown is present at a certain concentration. Therefore, the sterilization is preferably performed so that the yellowish brown of the *Lactobacillus* strain reaches at least a predetermined concentration. Specifically, the sterilization is preferably performed so that the brightness L* and chromaticity a* values of the L*a*b* color system calculated as follows are less than or equal to 65 and greater than or equal to -4, respectively.

The *Lactobacillus* sp. is sterilized in the culture broth after the cultivation, the culture medium is removed by subjecting the culture broth to centrifugation, membrane separation, or precipitation, and 5 g of a strain concentrate having a solid content of 4 to 7% by weight is prepared. Then, the brightness L* and chromaticity a* values are calculated when 5 g of the strain concentrate is added into a quartz glass Petri dish having a diameter of 35 mm, and a colorimetric color of the L*a*b* color system is measured for the strain concentrate per se. Also, the colorimetry may be performed using a colorimeter.

The *Lactobacillus* strain obtained according to one exemplary embodiment of the present invention may be subjected to atomization and reaggregation prevention processes. Therefore, the activity of the *Lactobacillus* strain, such as an ability of the *Lactobacillus* strain to induce IL-12 production, and the quality stability may be well maintained. Such a method may include a method disclosed in Japanese Patent No. 4621218. Specifically, the method may be performed as follows.

First, the *Lactobacillus* strain is pulverized or dispersed until the average particle size of the *Lactobacillus* strain reaches a nanometer (nm) size less than 1 micrometer (µm), preferably 0.6 µm. These pulverization and dispersion processes may be performed separately or simultaneously. Also, the corresponding particle size may be measured using a particle size distribution meter or an electron microscope to check whether the particle size is less than 1 µm.

An atomization method may include known techniques such as an agitator, a mixer, a ball mill, a bead mill, a jet mill, a homogenizer, a generator, a "nanomizer" (commercially available from Yoshida Machinery Co., Ltd.), an "ultimaizer system" (commercially available from Sugino Machine Co., Ltd.), etc., regardless of wet/dry techniques. In this case, a wet dispersion process is preferably performed on the *Lactobacillus* strain. For example, the *Lactobacillus* strain is included at a content of 2 to 30% by weight, more preferably 5 to 20% by weight, based on the calculated dry matter, and the atomization method may be performed by circulating a suspension whose pH value is adjusted to pH 6.0 to 7.0 at a condition of a pressure of 80 to 200 kg/cm², more preferably 100 to 170 kg/kg/cm² using a high-pressure homogenizer.

Next, the reaggregation prevention process is performed using a dispersing agent. Such a method preferably includes adding a dispersing agent to a *Lactobacillus* strain upon the atomization, atomizing the *Lactobacillus* strain, and pulverizing the resulting suspension, or atomizing the *Lactobacillus* strain, adding a dispersing agent to the *Lactobacillus* strain, and pulverizing the resulting suspension. The pulverization may be performed by spray-drying or freeze-drying the Lactobacillus strain after the atomization. Therefore, the suspension is pulverized in a state in which the dispersing agent is brought into contact with the atomized *Lactobacillus* strain. As a result, the *Lactobacillus* strain has good dispersibility even when resuspended in water, and the activity and quality stability of the *Lactobacillus* strain may be maintained well since the reaggregation of the atomized *Lactobacillus* strain is prevented.

Preferred examples of the dispersing agent that may be used herein may include polysaccharides such as dextrin, soluble plant fibers, and indigestible dextrin, low-molecular saccharides such as trehalose, lactose, and maltose, collagen, and peptides such as whey degradation products, and soy protein degradation products. The amount of the added dispersing agent is preferably in a range of 20 to 1,000 parts by weight, more preferably a range of 50 to 1,000 parts by weight, and most preferably a range of 100 to 600 parts by weight, based on 100 parts by weight of the calculated dry matter of the *Lactobacillus* strain. When the amount of the added dispersing agent is less than 20 parts by weight based on 100 parts by weight of the calculated dry matter of the *Lactobacillus* strain, an effect of adding the dispersing agent is insufficient. On the other hand, when the amount of the added dispersing agent is greater than 1,000 parts by weight, it is difficult to ensure the content of the *Lactobacillus* strain. Therefore, both of the two cases are not preferred.

Hereinafter, one use example of the *Lactobacillus* strain obtained according to one exemplary embodiment of the present invention will be described.

The *Lactobacillus* strain obtained according to one exemplary embodiment of the present invention has an excellent effect of inducing IL-12 production, and thus is highly suitable as an active ingredient of an IL-12 production inducer, as will be described in Examples below. In this case, the *Lactobacillus* strain subjected to the atomization and reaggregation prevention processes may be preferably used as it is, or the *Lactobacillus* strain dried using the spray or freeze drying process may be preferably used. On the other hand, when the atomized *Lactobacillus* strain is washed with water, components degraded into a low-molecular size by means of the atomization, or nutrient components exposed to the outside by means of the atomization may be washed out. Therefore, both of the two cases are not preferred.

For the IL-12 production inducer, other materials may be blended in addition to the *Lactobacillus* strain obtained according to one exemplary embodiment of the present invention, but the present invention is not particularly limited thereto. When necessary, a pharmaceutically available base or carrier may be added, and prepared into the form of, for example, a tablet, a granule, a capsule, a pill, a discutient, a solution, a powder, a jelly, and a candy, which may be used as an oral formulation. Also, the pharmaceutically available base or carrier may be prepared into the form of an ointment, a cream a gel, and a lotion, which may be used as a solution for external use on the skin.

For the IL-12 production inducer, a food source may be blended as another material in addition to the *Lactobacillus* strain obtained according to one exemplary embodiment of the present invention. For example, the food source may include a variety of sugars, an emulsifying agent, a sweetening agent, an acidulant, a fruit juice, a flavor, etc. More particularly, the food source may include saccharides such as glucose, sucrose, fructose, and honey, sugar alcohols such as sorbitol, xylitol, erythritol, lactitol, and palatinit, emulsifying agents such as sucrose fatty acid ester, glycerin fatty acid ester, and lecithin. In addition, a variety of vitamins such as vitamin A, vitamin B, vitamin C, and vitamin E, herbal extracts, grain ingredients, vegetable ingredients, milk ingredients, and the like may also be blended.

For the IL-12 production inducer, the content of the *Lactobacillus* strain may be properly determined in consideration of the relationship between the amount and the effective dose of the *Lactobacillus* strain when the IL-12 production inducer is prepared into various forms, but the present invention is not particularly limited. Typically, when the IL-12 production inducer is in a solid form, the IL-12 production inducer is preferably present at a content of 0.1 to 100% by weight, more preferably a content of 1 to 50% by weight, based on a total of the calculated dry matter of the *Lactobacillus* strain. Also, when the IL-12 production inducer is in a liquid or jelly form, the IL-12 production inducer is preferably present at a content of 0.1 to 50% by weight, more preferably a content of 1 to 5% by weight, based on a total of the calculated dry matter of the *Lactobacillus* strain.

In the case of the dosage form of the IL-12 production inducer, the IL-12 production inducer may be orally ingested to act in the body, or applied onto the skin. Also, a breathing apparatus may be used to inhale the IL-12 production inducer, but the dosage form is not particularly limited.

When an adult orally ingests the IL-12 production inducer, the dose of the IL-12 production inducer is not particularly limited, but may be typically in a range of approximately 0.01 to 10 g a day when calculated based on the dry matter of the *Lactobacillus* strain. The shapes or composition of these feed may be determined according to the description of use patterns when the feed is ingested orally.

Meanwhile, the *Lactobacillus* strain obtained according to one exemplary embodiment of the present invention may be blended with confectionery such cookies, senbei, jelly, sweet bean jelly, yogurt, and Deli Manjoo, foods such as soft drinks, nutritional drinks, and soups, and used. Also, the *Lactobacillus* strain may be blended with health foods provided for the purpose of maintaining or improving hygiene and health care as a positive meaning rather than typical food products. Also, the *Lactobacillus* strain may also be used as forage for livestock, racehorses, and ornamental pets; and forages for animals such as pet foods.

### EXAMPLES

Hereinafter, the present invention will be described in detail with reference to the following Examples. [Use strains]
Strains listed in Table 1 were used as the *Lactobacillus* strain.

**[Table 1]**

| Strains names | Abbreviations |
|---|---|
| *Lactobacillus plantarum* SNK12 (Accession No. NITE P-1445) | SNK12 |
| *Lactobacillus plantarum* nF1 (Accession No. NITE P-1462) | nF1 |
| *Lactobacillus plantarum* KH3 (Accession No. NITE AP-1476) | KH3 |
| *Enterococcus faecalis* KH2 (Accession No. NITE P-1444) | KH2 |
| *Lactobacillus brevis* (Accession No. FERM BP-4693) | Labre |
| *Lactococcus lactis sp cremoris* (Accession No. FERM P-20848) | CF4 |

### [Culturing of Lactobacillus strain]

The culturing of the Lactobacillus strains was performed as follows, except that it was especially noted.

A "Difco Lactobacilli MRS broth" (trade name, Becton Dickinson & Company, Japan) was used as the culture medium. When it was assumed that the Lactobacillus strains were cultured at a starting strain concentration of 1×10⁶ to 1×10⁷ cfu/mL, the culturing conditions were set until the final strain concentration reaches 5×10⁹ to 5×10¹⁰ cfu/mL when the pH of the culture broth was set in a range of pH 6.0 to 6.5 at the start of this cultivation, and the *Lactobacillus* strains were then cultivated while adjusting pH. The cultivation was performed at temperatures suitable for the growth of the respective Lactobacillus strains, that is, a temperature of 32°C on the *Lactobacillus* strains such as *L. plantarum* nF1, *L. plantarum* SNK12 and *L. plantarum* KH3, a temperature of 37°C on the *Lactobacillus* strain such as *E. faecalis* KH2, a temperature of 31 to 32°C on the *Lactobacillus* strain such as *L. brevis* FERP BP-4693, and a temperature of 30°C on the *Lactobacillus* strain such as *L. lactis sp. cremoris* CF4. When the pH of the culture broth was adjusted, the cultivation was performed while the pH of the culture broth was adjusted with caustic soda at a setting of pH ± 0.1 using a pH stat.

### [Post-cultivation process]

A post-cultivation process was performed as follows, except that it was especially noted.

90 mL of a culture broth was sterilized at 80°C for 10 minutes in an autoclave, and centrifuged to collect the strain. Then, the collected strain was suspended in approximately 20 mL of a phosphate buffer until the strain concentration reached 1 to 4%. The resulting suspension was thoroughly dispersed using a Teflon homogenizer to prevent the strains from aggregating to each other. The strain suspension thus obtained was sterilized at 121°C for 15 minutes, sonicated for 30 minutes to prevent the aggregation of the strains, and the solid content of the strain suspension was measured simultaneously or separately. Then, a IL-12 production induction test was performed by adjusting the strain suspension to a predetermined concentration, based on the measured solid content of the strain suspension.

### [IL-12 production induction test]

An *in vitro* culture system of mouse spleen cells was used in an IL-12 production induction test. Specifically, spleen cells were extracted from 8-week-old BALB/c mice, and a cell suspension (1.0×10⁶ cells/mL) was prepared in an RPMI1640 culture medium supplemented with 10% FBS, 10 µM 2-mercaptoethanol, 10 mM HEPES, 100 U/mL penicillin, and 100 µg/mL streptomycin according to a typical method. The *Lactobacillus* strain was added to the cell suspension until the final concentration reached 10 µg/mL when it was calculated as the dry matter of the *Lactobacillus* strain, and seeded to each well of a 96-well plate at an amount of 0.2 mL. The *Lactobacillus* strain was cultivated for 24 hours under conditions of a temperature of 37°C and 5% CO₂, and a culture supernatant was recovered after the cultivation to measure an amount of IL-12 in the culture supernatant. Also, a kit "EMIL12" (trade name, commercially available from Thermo Scientific) was used for measurements, and the results were calculated as an average of 6 wells of an immunoplate.

### Experimental Example 1

*L. plantarum* nF1 was used as the *Lactobacillus* strain, and cultivated while adding an alkaline chemical (caustic soda) to a culture medium to adjust pH (hereinafter referred to as "neutralization culture"), and also cultivated without adjusting the pH (hereinafter referred to as "stationary culture"). Thereafter, the abilities to induce IL-12 production were compared for both of the *Lactobacillus* strains. Also, the abilities to induce IL-12 production were also compared between the *Lactobacillus* strains obtained from the stationary culture after the neutralization culture. The results are listed in Table 2.

**[Table 2]**

| Strain types | Culture conditions | Final pH | Ability to induce IL-12 production (pg/mL) |
|---|---|---|---|
| nF1 | 1. Stationary culture for 20 Hrs | 3.8 | 1,800 |
| | 2. Stationary culture for 0 Hrs after neutralization culture for 20 Hrs | 6.1 | 50 |
| | 3. Stationary culture for 2 Hrs after neutralization culture for 20 Hrs | 5.5 | 700 |
| | 4. Stationary culture for 4 Hrs after neutralization culture for 20 Hrs | 5.0 | 1,300 |
| | 5. Stationary culture for 6 Hrs after neutralization culture for 20 Hrs | 4.9 | 1,400 |
| | 6. Stationary culture for 16 Hrs after neutralization culture for 20 Hrs | 7.8 | 1,550 |

As listed in Table 2, the *Lactobacillus* strain, *L. plantarum* nF1 having an ability to induce IL-12 production, obtained from the stationary culture at 32°C for 20 hours had an IL-12 production volume of 1,800 pg/mL. On the contrary, when the *Lactobacillus* strain was subjected to the neutralization culture in the same culture medium, the volume of the *Lactobacillus* strain increased by approximately three times, compared to when the *Lactobacillus* strain was subjected to the stationary culture, but the IL-12 production volume as the ability to induce IL-12 production was reduced to 50 pg/mL. On the other hand, when the neutralization culture was performed for 20 hours, a pH stat was turned off, and the stationary culture was performed, it was clearly revealed as a result that the ability to induce IL-12 production was enhanced as the stationary culture time was extended. Therefore, it was clearly revealed that a decrease in the ability to induce IL-12 production by means of the neutralization culture was recovered when the stationary culture was performed after the neutralization culture without controlling the pH.

### Experimental Example 2

The same test as in Experimental Example 1 was performed on the *Lactobacillus* strain, *L. brevis* FERP BP-4693. The results are listed in Table 3.

**[Table 3]**

| Strain types | Culture conditions | Final pH | Ability to induce IL-12 production (pg/mL) |
|---|---|---|---|
| Labre | 1. Stationary culture for 29 Hrs | 3.8 | 783 |
| | 2. Stationary culture for 0 Hrs after neutralization culture for 23 Hrs | 6.1 | 152 |
| | 3. Stationary culture (at 30°C) for 60 Hrs after neutralization culture for 23 Hrs | 4.7 | 439 |
| | 4. Stationary culture (at 40°C) for 60 Hrs after neutralization culture for 23 Hrs | 4.5 | 897 |

As shown in Table 3, the *Lactobacillus* strain, *L. brevis* FERP BP-4693 having an ability to induce IL-12 production, obtained from the stationary culture at 31°C for 29 hours had an IL-12 production volume of 7,83 pg/mL. On the contrary, when the *Lactobacillus* strain was subjected to the neutralization culture in the same culture medium, the volume of the *Lactobacillus* strain increased by approximately three times, compared to when the *Lactobacillus* strain was subjected to the stationary culture, but the IL-12 production volume as the ability to induce IL-12 production was reduced to 152 pg/mL. On the other hand, when the neutralization culture was performed for 23 hours, a pH stat was turned off, and the stationary culture was performed, it was clearly revealed as a result that the ability to induce IL-12 production was enhanced as the stationary culture time was extended. Therefore, it was clearly revealed that a decrease in the ability to induce IL-12 production by means of the neutralization culture was recovered when the stationary culture was performed after the neutralization culture without controlling the pH, regardless of the type of the *Lactobacillus* strain.

### Experimental Example 3

From the results of Experimental Examples 1 and 2, it was revealed that the ability to induce IL-12 production was enhanced when the pH adjustment was suspended after the neutralization culture, and the stationary culture was then performed. Accordingly, it was tested whether the ability to induce IL-12 production was further enhanced by keeping the corresponding strains under severe conditions upon the stationary culture. Also, *L. plantarum* SNK12 was used as the *Lactobacillus* strain. The results are listed in Table 4.

**[Table 4]**

| Strain types | Culture conditions | Final pH | Ability to induce IL-12 production (pg/mL) |
|---|---|---|---|
| SNK12 | 1. Stationary culture for 24 Hrs | 3.7 | 1,050 |
| | 2. Stationary culture for 0 Hrs after neutralization culture for 20 Hrs | 6.1 | 50 |
| | 3. Stationary culture for 4 Hrs after neutralization culture for 20 Hrs | 5.0 | 1,100 |
| | 4. Stationary culture (at 40°C) for 4 Hrs after neutralization culture for 20 Hrs | 4.9 | 1,400 |
| | 5. Stationary culture for 4 Hrs (in the presence of 1% NaCl) after neutralization culture for 20 Hrs | 5.1 | 1,250 |
| | 6. Stationary culture for 4 Hrs (in the presence of 2% NaCl) after neutralization culture for 20 Hrs | 5.2 | 1,750 |
| | 7. Stationary culture at 40°C for 4 Hrs (in the presence of 2% NaCl) after neutralization culture for 20 Hrs | 5.1 | 1,850 |

As listed in Table 4, the *Lactobacillus* strain, *L. plantarum* SNK12 having an ability to induce IL-12 production, obtained from the stationary culture at 32°C for 24 hours had an IL-12 production volume of 1,050 pg/mL. On the contrary, when the *Lactobacillus* strain was subjected to the neutralization culture for 20 hours in the same culture medium, the volume of the *Lactobacillus* strain increased by approximately three times, compared to when the *Lactobacillus* strain was subjected to the stationary culture, but the IL-12 production volume as the ability to induce IL-12 production was reduced to 50 pg/mL. On the other hand, when the neutralization culture was performed for 20 hours, a pH stat was turned off, and the stationary culture was performed for 4 hours, the IL-12 production volume increased to 1,100 pg/mL, which resulted in recovery of the ability to induce IL-12 production. Also, it was clearly revealed that the ability to induce IL-12 production was further enhanced when a saline solution was added to the culture medium upon the stationary culture, or the *Lactobacillus* strain, *L. plantarum* SNK12, was heated to a severe culture temperature of 40°C to propagate the *Lactobacillus* strain. An effect of activating the ability to induce IL-12 production was shown to be highest when the stationary culture, the addition of saline solution, and the high temperature were combined.

From these results, it was clearly revealed that the ability of the *Lactobacillus* strain to induce IL-12 production was recovered or further enhanced when the corresponding strain was forcedly kept under the severe stress conditions for growth. Therefore, it was clearly revealed that a sufficient volume of the strain was obtained by applying stress after the neutralization culture, and the *Lactobacillus* strain having a high ability to induce IL-12 production was able to be prepared as well.

### Experimental Example 4

It is necessary to prevent the self-digestion of the *Lactobacillus* strain when the *Lactobacillus* strain is supplied as a preparation so as to secure the stability. As the process of sterilizing and collecting the strain after the cultivation is completed, two methods, that is, a method of removing the culture medium components by means of an MF membrane or centrifugation and sterilizing a concentrate of the *Lactobacillus* strain which is suspended in a medium such as water or a buffer, and a method of sterilizing the *Lactobacillus* strain in advance in a state in which a culture broth is left after the cultivation and collecting the *Lactobacillus* strain can be considered. Among these, it was examined which method was to be adopted. For the examination, the effects when the culture broth was sterilized at 80°C for 10 minutes after the cultivation were compared to those when the *Lactobacillus* strain was collected by centrifugation, suspended in a phosphate buffer, and sterilized at the same conditions. The results are listed in Table 5.

**[Table 5]**

| Strain types | Culture and sterilization conditions | Ability to induce IL-12 production (pg/mL) |
|---|---|---|
| nF1 | Stationary culture for 6 Hrs, collection and sterilization after neutralization culture for 27 Hrs | 200 |
| | Stationary culture for 6 Hrs, sterilization and collection after neutralization culture for 27 Hrs | 1,000 |
| Labre | Stationary culture for 6 Hrs, collection and sterilization after neutralization culture for 40 Hrs | 120 |
| | Stationary culture for 6 Hrs, sterilization and collection after neutralization culture for 40 Hrs | 580 |
| SNK2 | Stationary culture for 6 Hrs, collection and sterilization after neutralization culture for 20 Hrs | 50 |
| | Stationary culture for 6 Hrs, sterilization and collection after neutralization culture for 20 Hrs | 1,100 |
| KH2 | Stationary culture for 6 Hrs, collection and sterilization after neutralization culture for 17 Hrs | 1,300 |
| | Stationary culture for 6 Hrs, sterilization and collection after neutralization culture for 17 Hrs | 2,100 |
| CF4 | Stationary culture for 6 Hrs, collection and sterilization after neutralization culture for 18 Hrs | 275 |
| | Stationary culture for 6 Hrs, sterilization and collection after neutralization culture for 18 Hrs | 738 |

As listed in Table 5, when any strain was sterilized in the culture broth after the cultivation, and then collected, the ability to induce IL-12 production was significantly higher, compared to when any strain was collected and then sterilized in a state in which the culture broth was removed. Also, when any strain was sterilized and collected, the *Lactobacillus* strain turned yellowish brown thickly, compared to when any strain was collected and sterilized. The reasons for these results being obtained were not clear, but one possibility was that any of physiological responses appeared in the *Lactobacillus* strain when the *Lactobacillus* strain was sterilized in the culture broth after the cultivation rather than when the *Lactobacillus* strain was collected and sterilized.

### Experimental Example 5

From the results obtained in Experimental Example 4, it was revealed that, when the *Lactobacillus* strain was sterilized in the culture broth after the cultivation, and then collected, the *Lactobacillus* strain had a higher ability to induce IL-12 production, compared to when the *Lactobacillus* strain was collected, and then sterilized in a state in which the culture broth was removed. Also, it was revealed that, when the strain was sterilized and collected, the *Lactobacillus* strain turned yellowish brown thickly, compared to when the strain was collected and sterilized. Accordingly, the relationship between the staining of the *Lactobacillus* strain and the ability to induce IL-12 production was further examined. Also, *L. plantarum* KH3 was used as the *Lactobacillus* strain, subjected to neutralization culture for 18 hours, followed by stationary culture for 6 hours. Thereafter, the *Lactobacillus* strain was sterilized by heating under the corresponding condition after the cultivation.

The staining of the *Lactobacillus* strain was subjected to colorimetry using a colorimeter (SQ-300H commercially available from Nippon Denshoku Industries Co., Ltd.), as follows.

90 mL of a culture broth was taken after sterilization, and divided to 50 mL centrifuge tubes at a volume of 45 mL, and then centrifuged at 8,400 rpm for 5 minutes to collect the strain. The strain was suspended in a phosphate buffer, homogenized using a Teflon homogenizer, and then re-centrifuged to remove the culture medium components. The resulting precipitate was suspended in a phosphate buffer, and then homogenized using a Teflon homogenizer to finally prepare the *Lactobacillus* strain at a volume of 25 mL. The strain suspension having a solid content of 4% thus prepared was transferred to a quartz glass Petri dish (diameter: 35 mm), and the brightness L* value and chromaticity a* and b* values of the L*a*b* color system were measured in this situation using a colorimeter (SQ-300H commercially available from Nippon Denshoku Industries Co., Ltd.). Also, Fig. 1 shows an image of the strain suspension.

The results are summarized, and listed in Table 6.

**[Table 6]**

| Strain types | Sterilization conditions | L* value | a* value | b* value | Ability to induce IL-12 production (pg/mL) |
|---|---|---|---|---|---|
| KH3 | 1. Not sterilized | 66.18 | -6.23 | 15.61 | 622 |
| | 2. At 60°C for 10 minutes | 69.67 | -6.60 | 20.08 | 430 |
| | 3. At 70°C for 10 minutes | 70.05 | -5.81 | 17.61 | 317 |
| | 4. At 80°C for 10 minutes | 60.42 | -3.07 | 19.58 | 1,237 |
| | 5. At 90°C for 10 minutes | 57.41 | -1.84 | 18.78 | 939 |
| | 6. At 100°C for 10 minutes | 56.31 | -0.53 | 19.40 | 585 |
| | 7. At 121°C for 10 minutes | n.d. | n.d. | n.d. | n.d. |

| | | | | | |
|---|---|---|---|---|---|
| n.d.: not determined | | | | | |

As listed in Table 6, the *Lactobacillus* strain has a higher ability to induce IL-12 production when the strain was sterilized under predetermined conditions after the cultivation, compared to when the strain was not sterilized. It was more preferred that the sterilization temperature was in a range of 80 to 100°C, and the improved ability to induce IL-12 production was very well correlated to a decrease in the brightness L* value and an increase in the chromaticity a* value of the strain.

## Claims

1. A method for preparing a Lactobacillus sp. having an ability to induce IL-12 production, the method comprising:
adding an alkaline chemical to a culture medium to adjust pH;
wherein the alkaline chemical is selected from sodium hydroxide, potassium hydroxide, calcium hydroxide or ammonia;
applying stress at a later stage of the cultivation;
wherein the stress is selected from the group consisting of [(a) and (c)], or [(a), (b) and (c)] :
(a) cultivating the Lactobacillus sp. without adding the alkaline chemical at pH 5 or less;
(b) cultivating the Lactobacillus sp. in a temperature range in which the propagation of the Lactobacillus sp. is suppressed;
(c) cultivating the Lactobacillus sp. after a salt is added at a concentration of 1% by weight or more in a culture broth; and
wherein the Lactobacillus sp. is a microorganism belonging to *Lactobacillus plantarum* or *Lactobacillus brevis*
and wherein the Lactobacillus sp. is sterilized at 80 °C to 100 °C for 5 to 20 minutes in the culture broth after the cultivation, and then collected.

2. The method of claim 1, wherein the Lactobacillus sp. is isolated from a plant.

3. The method of anyone of the claims 1 to 2, wherein the corresponding sterilization is performed so that when 5 g of strain concentrate having a solid content of 4 to 7% by weight prepared by Sterilizing the Lactobacillus after cultivation and Removing culture medium by centrifugation, membrane separation, or precipitation of the culture broth is added into a quartz glass Petri dish having a diameter of 35 mm,
the brightness L* value is less than or equal to 65 and a chromaticity a* value is greater than or equal to -4 as measured by a colorimetric color of the L*a*b* color system, wherein the Lactobacillus sp. is sterilized at 80 °C to 100° for 10 minutes.

4. A Lactobacillus sp. having an ability to induce IL-12 production, which is obtained by the method according to any one of the claims 1 to 3, wherein the sterilization is performed so that the brightness L* and chromaticity a* values of the L*a*b* color system are less than or equal to 65 and greater than or equal to -4, respectively measured by using a colorimeter, wherein the Lactobacillus sp. is sterilized at 80 °C to 100° for 10 minutes.

5. A Lactobacillus sp. of claim 4, wherein the Lactobacillus sp. is pulverized or dispersed until the average particle size of the Lactobacillus sp. reaches a size less than 1 micrometer (µm), preferably 0.6 µm.

## Patentansprüche

1. Verfahren zum Herstellen eines Lactobacillus sp. mit Fähigkeit zur Induzierung der IL-12-Produktion, wobei das Verfahren Folgendes umfasst:
Zusetzen einer alkalischen Chemikalie zu einem Kulturmedium, um den pH-Wert einzustellen;
wobei die alkalische Chemikalie aus Natriumhydroxid, Kaliumhydroxid, Calciumhydroxid oder Ammoniak ausgewählt ist;
Anwenden von Stress zu einem späteren Zeitpunkt der Kultivierung;
wobei der Stress aus der Gruppe bestehend aus [(a) und (c)] oder [(a), (b) und (c)] ausgewählt ist:
(a) Kultivieren des Lactobacillus sp. ohne Zusatz der alkalischen Chemikalie bei pH 5 oder darunter;
(b) Kultivieren des Lactobacillus sp. in einem Temperaturbereich, in dem die Ausbreitung des Lactobacillus sp. unterdrückt wird;
(c) Kultivieren des Lactobacillus sp. nachdem ein Salz in einer Konzentration von 1 Gew.-% oder mehr in eine Kulturbouillon gegeben wurde; und
wobei das Lactobacillus sp. ein Mikroorganismus ist, der zu *Lactobacillus plantarum* oder *Lactobacillus brevis* gehört
und wobei das Lactobacillus sp. nach der Kultivierung 5 bis 20 Minuten bei 80 °C bis 100 °C in der Kulturbouillon sterilisiert und dann gewonnen wird.

2. Verfahren nach Anspruch 1, wobei das Lactobacillus sp. aus einer Pflanze isoliert wird.

3. Verfahren nach einem der Ansprüche 1 bis 2, wobei die entsprechende Sterilisierung durchgeführt wird, so dass, wenn 5 g Stammkonzentrat, das einen Feststoffgehalt von 4 bis 7 Gew.-% aufweist, hergestellt durch Sterilisieren des Lactobacillus nach der Kultivierung und Entfernen des Kulturmediums durch Zentrifugation, Membrantrennung oder Ausfällung der Kulturbouillon, in eine Quarzglas-Petrischale mit einem Durchmesser von 35 mm gegeben wird,
der Helligkeitswert L* kleiner oder gleich 65 ist und ein Chromatizitätswert a* größer oder gleich -4 ist, gemessen durch eine kalorimetrische Farbe des L*a*b*-Farbsystems, wobei das Lactobacillus sp. 10 Minuten bei 80 °C bis 100 °C sterilisiert wird.

4. Lactobacillus sp. mit Fähigkeit zur Induzierung der IL-12-Produktion, das durch das Verfahren nach einem der Ansprüche 1 bis 3 erhalten wird, wobei die Sterilisation derart durchgeführt wird, dass der Helligkeitswert L* und der Chromatizitätswert a* des L*a*b*-Farbsystems kleiner oder gleich 65 bzw. größer oder gleich -4 sind, gemessen unter Verwendung eines Kolorimeters, wobei das Lactobacillus sp. 10 Minuten bei 80 °C bis 100 °C sterilisiert wird.

5. Lactobacillus sp. nach Anspruch 4, wobei das Lactobacillus sp. pulverisiert oder dispergiert wird, bis die durchschnittliche Partikelgröße des Lactobacillus sp. eine Größe von weniger als 1 Mikrometer (µm), vorzugsweise 0,6 µm erreicht.

## Revendications

1. Procédé pour préparer un Lactobacillus sp. apte à induire la production d'IL-12, le procédé comprenant :
l'ajout d'un produit chimique alcalin à un milieu de culture pour ajuster le pH ;
dans lequel le produit chimique alcalin est choisi parmi l'hydroxyde de sodium, l'hydroxyde de potassium, l'hydroxyde de calcium ou l'ammoniac ;
l'application d'une contrainte à un stade ultérieur de la culture ;
dans lequel la contrainte est choisie dans le groupe constitué de [(a) et (c)], ou [(a), (b) et (c)] :
(a) la culture du Lactobacillus sp. sans ajouter le produit chimique alcalin à un pH 5 ou moins ;
(b) la culture du Lactobacillus sp. dans une plage de température dans laquelle la propagation du Lactobacillus sp. est supprimée ;
(c) la culture du Lactobacillus sp. après l'ajout d'un sel à une concentration de 1 % en poids ou plus dans un bouillon de culture ; et
dans lequel le Lactobacillus sp. est un microorganisme appartenant à *Lactobacillus plantarum* ou à *Lactobacillus brevis*
*et* dans lequel le Lactobacillus sp. est stérilisé à 80 °C jusqu'à 100 °C pendant 5 à 20 minutes dans le bouillon de culture après la culture, puis collecté.

2. Procédé selon la revendication 1, dans lequel le Lactobacillus sp. est isolé d'une plante.

3. Procédé selon l'une quelconque des revendications 1 et 2, dans lequel la stérilisation correspondante est effectuée de sorte que lorsque 5 g de concentré de souche ayant une teneur en matière sèche de 4 à 7 % en poids sont préparés en stérilisant le Lactobacillus après culture et en retirant le milieu de culture par centrifugation, la séparation par membrane ou la précipitation du bouillon de culture est ajoutée dans une boîte de Pétri en verre de quartz ayant un diamètre de 35 mm, la valeur de luminosité L* est inférieure ou égale à 65 et une valeur de chromaticité a* est supérieure ou égale à -4 telle que mesurée par une couleur colorimétrique du système de couleurs L*a*b*, dans lequel le Lactobacillus sp. est stérilisé à 80 °C jusqu'à 100 °C pendant 10 minutes.

4. Lactobacillus sp. apte à induire la production d'IL-12, qui est obtenue par le procédé selon l'une quelconque des revendications 1 à 3, dans lequel la stérilisation est effectuée de sorte que les valeurs de luminosité L* et de chromaticité a* du système de couleur L*a*b* sont inférieures ou égales à 65 et supérieures ou égales à -4, mesurées respectivement à l'aide d'un colorimètre, dans lequel le Lactobacillus sp. est stérilisé à 80 °C jusqu'à 100 °C pendant 10 minutes.

5. Lactobacillus sp. selon la revendication 4, dans lequel le Lactobacillus sp. est pulvérisé ou dispersé jusqu'à ce que la taille moyenne des particules du Lactobacillus sp. atteigne une taille inférieure à 1 micromètre (µm), de préférence 0,6 µm.
